# EUROPÄISCHE PATENTSCHRIFT

(11) **EP 1 691 712 B1**
(45) Veröffentlichungstag und Bekanntmachung des Hinweises auf die Patenterteilung: **11.06.2008**
(21) Anmeldenummer: 04803633.9
(22) Anmeldetag: 08.12.2004
(51) Int. Cl.: A61C 13/00

(54) **VERFAHREN ZUR ERMITTLUNG DER FORM EINES RESTZAHNBEREICHS**
METHOD FOR DETERMINING THE SHAPE OF A RESIDUAL TOOTH ZONE
PROCEDE POUR DETERMINER LA FORME D'UNE ZONE DE DENT RESIDUELLE

(30) Priorität: 09.12.2003 DE 10357699
(43) Veröffentlichungstag der Anmeldung: 23.08.2006
(73) Patentinhaber: DeguDent GmbH, 63457 Hanau-Wolfgang (DE)
(72) Erfinder: VÖLKL, Lothar, 63773 Goldbach (DE); VON SCHROETER, Philip, 63517 Rodenbach (DE)
(74) Vertreter: Stoffregen, Hans-Herbert
(86) Internationale Anmeldenummer: PCT/EP2004/013964
(87) Internationale Veröffentlichungsnummer: WO 2005/058183

(56) Entgegenhaltungen:
- EP-A- 0 913 130
- WO-A-01/64128
- US-A- 4 767 330
- US-A- 5 338 198
- US-A- 5 605 459

## Beschreibung

Die Erfindung bezieht sich auf ein Verfahren zur Ermittlung der Form eines mit einer zahnärztlichen Restauration wie Brücke oder Gerüst zu versehenden Duplikats eines Restzahnbereichs, wobei mit der Restauration zu versehende und/oder ihre Gestaltung bestimmende Duplikatsabschnitte aus dem Duplikat gelöst und deren Formen zuzuordnende Formdaten ermittelt und in einem Rechner gespeichert werden, mittels der unter Berücksichtigung räumlicher Zuordnung der Duplikatsabschnitte zueinander die Form der Restauration berechnet wird.

Ein entsprechendes Verfahren ist der EP 0 731 673 B1 (WO 96/10371) zu entnehmen. Dabei ist vorgesehen, dass in einem ersten Verfahrensschritt gegenseitige Positionen der Duplikatsabschnitte in dem Duplikat mittels eines Nivellierapparates bestimmt werden. In einem zweiten Schritt werden die aus dem Duplikat herausgenommenen Duplikatsabschnitte mittels einer Abtasteinheit abgetastet, um Konturdaten zu ermitteln.

Schließlich werden in einem dritten Schritt die Konturdaten mit den Positionsdaten verknüpft.

Ein diesbezügliches Verfahren ist aufgrund der notwendigen Hardware aufwendig und birgt somit eine Vielzahl von Fehlerquellen.

Aus der EP-B-0 913 130 ist ein Verfahren zur Herstellung eines Zahnersatzteils bekannt, bei dem zunächst die Form eines Duplikats ermittelt, das Duplikat sodann zertrennt, Formen von herausgelösten Duplikatsabschnitten ermittelt und schließlich die Daten des Duplikats und die der Duplikatsabschnitte zusammengeführt werden. Ein diesbezügliches Verfahren führt zu genauen Messergebnissen, bedarf jedoch neben einer aufwendigen Software, aufwendige technische Mittel, um zum einen das Duplikat und zum anderen die Duplikatsabschnitte zu scannen. Hierzu sind gesonderte Einspannvorrichtungen vorgesehen, die auf einen Scanner auszurichten sind.

Der vorliegenden Erfindung liegt das Problem zu Grunde, ein Verfahren der eingangs genannten Art so auszubilden, dass mit hoher Genauigkeit die Form des mit der Restauration zu versehenden bzw. die Gestaltung der Restauration bestimmenden Restzahnbereichs und damit die Form der Restauration selbst ermittelt werden kann. Gleichzeitig sollen apparativ bedingte Messfehler minimiert werden:

Zur Lösung des Problems sieht die Erfindung im Wesentlichen vor, dass die entnommenen Duplikatsabschnitte zueinander nach einer in dem Rechner abgelegten und vom Duplikat unabhängigen Referenzierung individuell referenziert werden und dass diese Daten zur Ermittlung der Form der zahnärztlichen Restauration in dem Rechner mit den Formdaten der Duplikatsabschnitte verknüpft werden. Mit anderen Worten sieht die Erfindung vor, dass die Duplikatsabschnitte in ihrer räumlichen Zuordnung zueinander nach einer in dem Rechner abgelegten Referenzierung individuell referenziert sind oder werden.

Referenzieren unabhängig vom Duplikat bedeutet dabei, dass nicht die Geometrie des Duplikats, das durch den individuellen Abdruck des mit einem Zahnersatz zu versehenen Restzahn- bzw. Kieferbereichs gewonnen und nach der EP-B-0 913 130 zwingend als Referenzierung an sich benutzt wird; sondern dass eine unabhängig von der individuellen Geometrie des Duplikats festgelegte bzw. vorgegebene Referenzierung, die in dem Rechner abgelegt ist, benutzt wird, um die Duplikatsabschnitte räumlich und geometrisch einander zuzuordnen. Dies schließt jedoch nicht aus, dass bereits bei der Herstellung des Duplikats bzw. bei der Abdrucknahme durch die Verwendung z. B. eines referenzierenden Abdrucklöffels eine gewünschte Referenzierung unabhängig von dem individuellen Verlauf des Duplikats des Restzahn- bzw. Kieferbereichs aufgeprägt wird.

Abweichend vom vorbekannten Stand der Technik ist es nicht mehr erforderlich, das Duplikat vollständig abzutasten oder einen Nivellierapparat zu verwenden, um die Position der Duplikatsabschnitte zueinander zu bestimmen, die mit der zahnärztlichen Restauration versehen werden sollen. Vielmehr ist es einzig und allein erforderlich, dass das Duplikat referenziert wird, um sodann unter Berücksichtigung dieser Referenzierung die Form der Duplikatsabschnitte durch z. B. Scannen zu bestimmen, wobei die Referenzierung die räumliche Zuordnung der Duplikatsabschnitte vorgibt, also aus der Referenzierung die räumliche Zuordnung ermittelt wird.

Die Referenzierung erfolgt nicht durch das Duplikat selbst, wie dies nach dem Stand der Technik der Fall ist. Vielmehr wird das Duplikat vorzugsweise zu einer Aufnahme wie Halterung, insbesondere einer das Duplikat aufnehmende Grundplatte ausgerichtet, die ihrerseits referenziert ist. Somit wird eine vorhandene Referenzierung benutzt, die eine eindeutige geometrische Beziehung zwischen den Duplikatsabschnitten sicherstellt.

Der Referenzierung entsprechende Daten sind in dem Rechner abgelegt, so dass folglich als neue Daten nur diejenigen einzugeben sind, die der Form der Duplikatsabschnitte entsprechen. Somit erfolgt eine softwaremäßige Vereinfachung. Auch apparativ ergeben sich Vorteile dadurch, dass es nur noch einer Halteeinrichtung bzw. einer Art einer Halteeinrichtung zum Erfassen der jeweiligen Duplikatsabschnitte bedarf, die auf einen Sensor wie Scanner ausgerichtet wird. Üblicherweise wird hierzu der Duplikatsabschnitt in einen sogenannten Haltetopf aufgenommen, der eine Silikonmasse enthalten kann, in der der zu messende Duplikatsabschnitt fixierbar ist.

Insbesondere ist zur Referenzierung vorgesehen, dass das Duplikat auf einer Referenzen aufweisenden Grundplatte befestigt wird, die mit dem Duplikat zertrennt wie zersägt wird. Die einzelnen Grundplattenabschnitte weisen dabei die erforderlichen Referenzen auf, um eine geometrische bzw. räumliche Zuordnung der getrennt voneinander zu scannenden Duplikatsabschnitte sicherzustellen.

Dabei ist insbesondere vorgesehen, dass das Duplikat unterseitig plangeschliffen wird, um sodann auf der eine plane Oberfläche aufweisenden Grundplatte befestigt zu werden.

Insbesondere zeichnet sich die Erfindung durch die Verfahrensschritte aus:
- Abdrucknahme von zumindest einem zu versorgenden Restzahnbereich umfassenden Teil des Kiefers,
- Herstellen des Duplikats vorzugsweise durch Ausgießen des Abdrucks mit Gips,
- Befestigen des Duplikats auf der die Referenzen aufweisenden Grundplatte (zahntechnisches Modell),
- Zertrennen des zahntechnischen Modells zur Gewinnung von Modellabschnitten, die die Duplikatsabschnitte aufweisen,
- Vermessen der Duplikatsabschnitte zur Gewinnung von Formdaten und auf den jeweiligen Grundplattenabschnitten vorhandener Referenzen,
- Matchen von Daten, die den Referenzen der einzelnen Modellabschnitte entsprechen, mit den in dem Rechner abgelegten Daten der Referenzierung und
- Herstellen der zahntechnischen Restauration unter Berücksichtigung der Formdaten und der durch Matchen gewonnenen Daten.

Mit anderen Worten wird durch das Erfassen der aus den auf den einzelnen Modellabschnitten vorhandenen Referenzen gewonnenen Daten mit den Daten der Referenzierung die räumliche Zuordnung der Duplikatsabschnitte ermittelt. Diese Daten werden sodann mit den Formdaten, also den den Formen der einzelnen Duplikatsabschnitte entsprechenden Daten verknüpft, um die zahntechnische Restauration herstellen zu können, wie dies z. B. prinzipiell in der WO 99/47065 oder der WO 03/007834 offenbart ist.

Insbesondere ist vorgesehen, dass als Referenzen geometrische Eigenschaften der Grundplatte und/oder Markierungen dieser verwendet werden. Als Markierungen kommen Punkte oder Linien auf der Grundplatte in Frage. Als geometrische Eigenschaften sind bevorzugterweise Begrenzungen wie Kanten oder Flächen der Grundplatte zu nennen.

Insbesondere sollten Innen- und/oder Außenlängsseitenwandung der Grundplatte eine Zickzack- oder Wellengeometrie aufweisen, wobei die entsprechende Geometrie derart gewählt ist, dass eine eindeutige Zuordnung von Grundplattenabschnitten zueinander sichergestellt ist. So kann bei einer Zickzack-Geometrie die Höhe der Erhebung bzw. der Abstand dieser im erforderlichen Umfang variiert werden. Gleiches gilt bei einer Wellengeometrie.

Auch können bei einer Zickzack-Geometrie die jeweiligen Spitzen als Referenzpunkte verwendet werden, auch dann, wenn diese zum Beispiel beschädigt werden, sofern die die Spitzen schneidenden Begrenzungsflächen der Grundplatte ebenfalls eine eindeutige geometrische Lage zueinander aufweisen, wodurch eine Referenzierung erfolgt.

Um sicherzustellen, dass durch das auf die Grundplatte aufzubringende Duplikat im Randbereich der Grundplatte vorhandene Referenzen nicht abgedeckt werden, sollte das Duplikat zum Innen- und Außenrand der Grundplatte beabstandet sein.

Eine Referenzierung kann aber auch unmittelbar bei der Abdrucknahme erfolgen. So kann z. B. ein Abdrucklöffel derart gestaltet sein, dass sich Referenzen am Abdruck ergeben. Andere entsprechende Maßnahmen sind gleichfalls möglich. So können auch Referenzen unmittelbar auf den Abdruck aufgebracht werden.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung eines der Zeichnung zu entnehmenden bevorzugten Ausführungsbeispiels.

Es zeigen:
- Fig. 1: eine Prinzipdarstellung eines Duplikats,
- Fig. 2: eine das Duplikat nach Fig. 1 aufnehmende Grundplatte,
- Fig. 3: ein zahntechnisches Modell bestehend aus Duplikat nach Fig. 1 und Grundplatte nach Fig. 2,
- Fig. 4: eine Form zur lagerichtigen Aufnahme von Abschnitten des zahntechnischen Modells nach Fig. 3 und
- Fig. 5: eine Prinzipdarstellung einer einen Abschnitt des zahntechnischen Modells gemäß Fig. 3 aufnehmenden Halterung mit dieser zugeordnetem Sensor.

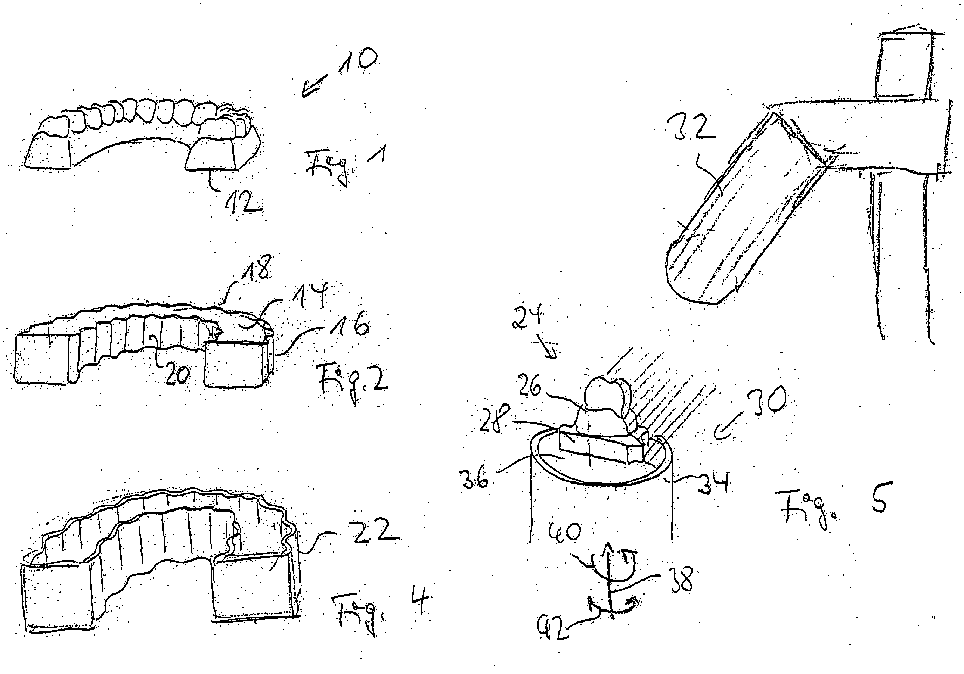

In Fig. 1 ist ein als Duplikat 10 bezeichnetes Positivmodell einer Situation im Mund eines Patienten dargestellt. Das Duplikat 10 entspricht somit einem Modell eines Restzahnbereichs, der mit einer zahnärztlichen Restauration wie Brücke oder Gerüst zu versehen ist. Hierzu wird ausgehend von einer zahnärztlichen Präparation zumindest von mit der Restauration zu versehenden Zahnstümpfen in gewohnter Weise ein Abdruck gefertigt, der dem Negativmodell der Situation im Mund des Patienten entspricht. Dabei soll das Negativmodell nicht nur die Zahnstümpfe, sondern auch die der Nachbarzähne bzw. deren Aproximalflächen umfassen. Von der Abformung bzw. dem Abdruck wird ein Positivmodell hergestellt, das üblicherweise aus Gips besteht.

Das Duplikat 10 wird sodann unterseitig (Fläche 12) plangeschliffen, um auf einer planen Fläche 14 einer konfektionierten Grundplatte 16 befestigt wie geklebt zu werden. Dabei zeigt die Grundplatte 16 eine Geometrie eines Bogenverlaufs, also eine solche, die einem Kiefer entspricht. Gegebenenfalls können zur einfachen Anpassung von individuellen Duplikaten 10 mehrere Größen von Grundplatten 16 zur Verfügung gestellt werden.

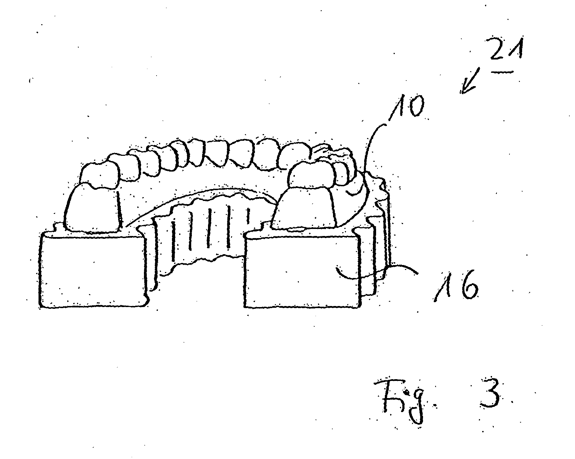

Unabhängig hiervon weist die Grundplatte 16 Referenzen auf, die eine Referenzierung ermöglichen bzw. bilden. Die Referenzen können insbesondere durch eine besondere Gestaltung der Grundplatte 16 vorgegeben sein. So ist im Ausführungsbeispiel ein besonders gestalteter Geometrieverlauf von Außenseitenwandung 18 bzw. Innenseitenwandung 20 bzw. deren Flächen vorgesehen, die Referenzen bilden. Dabei sind die Flächen 18, 20 derart gestaltet, dass jeder Abschnitt der Grundplatte 16 individuell referenziert, also gekennzeichnet ist mit der Folge, dass unter Berücksichtigung der jeweiligen Referenz eine eindeutige geometrische bzw. räumliche Zuordnung der entsprechenden Abschnitte der Grundplatte 16 zueinander sichergestellt ist. Durch die Befestigung des Duplikats 10 auf der Fläche 14 der Grundplatte 16 weisen folglich die Abschnitte des Duplikats 10 ebenfalls eine eindeutige räumliche Zuordnung zueinander auf. Das auf der Grundplatte 16 befestigte Duplikat 10 ist in Fig. 3 dargestellt. Die so hergestellte Einheit kann als zahntechnisches Modell 21 bezeichnet werden.

Nach Befestigen des Duplikats 10 auf der Grundplatte 16 werden aus dem Duplikat 10 zusammen mit der Grundplatte 16 Modellabschnitte 24 ausgeschnitten, die Einzelzähnen, unbezahnten Kieferabschnitten oder Zahnstümpfen entsprechen, die mit der zahntechnischen Restauration zu versehen sind bzw. deren Gestaltung bestimmen. Die Modellabschnitte 24 des zahntechnischen Modells 21 bestehen demzufolge aus einem Duplikatsabschnitt 26 und einem Abschnitt 28 der Grundplatte 16.

Der jeweils zu messende Modellabschnitt 24 wird in eine Halterung 30 eingesetzt, um mittels eines Sensors 32 wie Scanner die Geometrie zu erfassen und die entsprechenden Messwerte als digitale Daten einem Rechner zuzuführen, in dem der Referenzierung entsprechende Daten abgelegt sind. Somit ist es nur noch erforderlich, die Daten der Modellabschnitte 24 relativ zur im Rechner hinterlegten Referenzgetimetrie und damit zueinander auszurichten (matching), um einen Datensatz zu erthalten. Mit anderen Worten werden die der Referenzierung entsprechenden Daten mit denen aus den Referenzen der Modellabschnitte gewonnenen Daten gematcht, um eine räumliche Zuordnung der Duplikatabschnitte zu erreichen, die der im Duplikat und somit der Situation im Mund des Patienten entspricht. Neben den Positionsdaten des jeweiligen Modellabschnitts liegen auch Formdaten des auf dem jeweiligen Modellabschnitt befestigten Duplikatsabschnitts vor. Aus diesen Daten wird sodann die zahntechnische Restauration automatisch hergestellt, wie dies prinzipiell in der WO 99/47065 oder der WO 03/007834 offenbart ist. Auf die diesbezüglichen Offenbarungen wird somit ausdrücklich Bezug genommen.

Die Halterung 30 ist im Ausführungsbeispiel als Topf 34 ausgebildet, in dem sich eine Haltemasse wie Silikongummi 36 zur Aufnahme und Fixierung des Grundplattenabschnittes 28 und damit des Modellabschnitts 24 befindet. Der Topf 34 kann sodann um seine Längsachse 38 gedreht werden, wie durch die Pfeile 40, 42 symbolisiert wird. Hierdurch erfolgt im gewünschten Umfang eine Ausrichtung zu dem Sensor 32, um den Modellabschnitt 24 im erforderlichen Umfang erfassen bzw. scannen zu können. Dabei wird die Referenzierung, d. h. die Referenzpunkte des Grundplattenabschnitts 28 werden erfasst, um hierdurch eine eindeutige geometrische Zuordnung des jeweiligen erfassten Duplikatsabschnitts 26 zum Gesamtduplikat 10 bzw. die Position des Duplikatsabschnitts 26 in dem Duplikat 10 eindeutig ermitteln zu können.

Selbstverständlich besteht auch die Möglichkeit, den aus dem Grundplattenabschnitt 28 und dem Duplikatsabschnitt 26 bestehenden Modellabschnitt 24 zur Erfassung der Geometrie ergänzend entlang X- und Y-Richtung eines rechtwinkligen Koordinatensystems zu bewegen.

Mit anderen Worten kann eine Relativbewegung zwischen dem Modellabschnitt 24 und dem Sensor 32 um gewünschte Freiheitsgrade erfolgen. Auch kann der Sensor 32 gegebenenfalls in erforderlichem Umfang zu dem Abschnitt 24 ausgerichtet werden.

Zur lagerichtigen Ausrichtung der Modellabschnitte beim Aufpassen der hergestellten Restauration kann eine Form 22 verwendet werden, wie diese z. B. vom "Model-Tray"-System bekannt ist (Fig. 4).

Erfindungsgemäβ ist mit einfachen Maßnahmen eine Geometriebestimmung des Modells eines mit einem Zahnersatz wie Brücke zu versehenden Kieferbereichs gegeben.

## Patentansprüche

1. Verfahren zur Ermittlung der Form eines mit einer zahnärztlichen Restauration wie Brücke oder Gerüst zu versehenden Duplikats eines Restzahnbereichs, wobei mit der Restauration zu versehende und/oder ihre Gestaltung bestimmende Duplikatsabschnitte aus dem Duplikat gelöst und deren Formen zuzuordnende Formdaten ermittelt und in einem Rechner gespeichert werden, mittels der unter Berücksichtigung räumlicher Zuordnung der Duplikatsabschnitte zueinander die Form der Restauration berechnet wird,
**dadurch gekennzeichnet,**
**dass** die Duplikatsabschnitte in ihrer räumlichen Zuordnung zueinander nach einer im Rechner abgelegten und von dem Duplikat unabhängigen Referenzierung individuell referenziert sind oder werden.

2. Verfahren nach Anspruch 1,
**dadurch gekennzeichnet,**
**dass** zumindest von einem mit einer Restauration zu versorgenden Restzahnbereich des Kiefers ein Abdruck genommen wird, von dem Abdruck ein Modell als das Duplikat vorzugsweise durch Ausgießen des Abdrucks mit Gips hergestellt wird, das Duplikat auf einer konfektionierten und Referenzen aufweisenden Grundplatte befestigt wird, die Grundplatte mit dem auf dieser befestigten Duplikat zur Gewinnung der Duplikatsabschnitte zertrennt wird und sodann die Duplikatsabschnitte unter Berücksichtigung von Referenzen gemessen werden, die auf Grundplattenabschnitten vorhanden sind, auf denen die Duplikatabschnitte angeordnet sind.

3. Verfahren nach Anspruch 2,
**dadurch gekennzeichnet,**
das als Referenzen insbesondere geometrische Eigenschaften der Grundplatte und/oder Markierungen auf dieser verwendet werden.

4. Verfahren nach Anspruch 3,
**dadurch gekennzeichnet,**
**dass** als Markierungen Punkte und/oder Linien auf der Grundplatte verwendet werden.

5. Verfahren nach Anspruch 3,
**dadurch gekennzeichnet,**
**dass** als geometrische Eigenschaften Begrenzungen wie Kanten oder zumindest Flächenabschnitte der Grundplatte verwendet werden.

6. Verfahren nach zumindest Anspruch 2,
**dadurch gekennzeichnet,**
**dass** das Duplikat unterseitig plan geschliffen wird und auf einer planen Fläche der einem Zahnbogenverlauf folgenden Grundplatte befestigt wie geklebt wird.

7. Verfahren nach zumindest Anspruch 6,
**dadurch gekennzeichnet,**
**dass** das Duplikat derart auf der Grundplatte befestigt wird, dass das Duplikat allseitig zum Rand der Grundplatte beabstandet ist.

8. Verfahren nach zumindest Anspruch 2,
**dadurch gekennzeichnet,**
**dass** als Grundplatte eine solche verwendet wird, die in oder an zumindest einer entlang des Duplikats verlaufenden Längsseitenwandung eine Strukturierung wie Wellen- und/oder Zickzack-Geometrie aufweist.

9. Verfahren nach zumindest Anspruch 8,
**dadurch gekennzeichnet,**
**dass** als Referenzen Schnittpunkte oder virtuelle Schnittpunkte von Begrenzungsflächen der Wellen- und/oder Zickzack-Geometrie verwendet werden:

10. Verfahren nach zumindest Anspruch 1,
**gekennzeichnet durch** die Verfahrensabschnitte
- Abdrucknahme von zumindest einem zu versorgenden Restzahnbereich umfassenden Teil des Kiefers,
- Herstellen des Duplikats vorzugsweise **durch** Ausgießen des Abdrucks mit Gips,
- Befestigen des Duplikats auf der die Referenzen aufweisenden Grundplatte,
- Zertrennen der Grundplatte mit auf dieser befestigtem Duplikat zur Gewinnung von Modellabschnitten, die die Duplikatsabschnitte aufweisen,
- Vermessen der Duplikatsabschnitte zur Gewinnung von Formdaten und der auf den jeweiligen Grundplattenabschnitten vorhandenen Referenzen,
- Matchen von Daten, die den Referenzen der einzelnen Modellabschnitte entsprechen, mit den in dem Rechner abgelegten Daten der Referenzierung und
- Herstellen der zahntechnischen Restauration unter Berücksichtigung der Formdaten und der **durch** das Matchen gewonnenen Daten.

11. Verfahren nach Anspruch 1,
**dadurch gekennzeichnet,**
**dass** das Duplikat unmittelbar mit Referenzen versehen wird.

12. Verfahren nach Anspruch 11,
**dadurch gekennzeichnet,**
**dass** die Referenzen beim Herstellen eines Abdrucks erzeugt werden.

## Claims

1. Method for determining the form of a duplicate of a residual tooth area which is to be fitted with a dental restoration such as a bridge or framework, whereby the duplicate sections to be fitted with the restoration and/or duplicate sections determining their design are removed from the duplicate and their form data to be allocated to their forms is determined and stored in a computer, by means of which the form of the restoration is calculated taking into consideration the spatial allocation of the duplicate sections to each other,
**characterized in**
**that** the duplicate sections are or are being individually referenced as to their spatial allocation to each other according to a referencing stored in the computer, and which is independent of the duplicate.

2. Method according to claim 1,
**characterized in**
**that** a casting is taken from at least one of the residual teeth areas of the jaw to be fitted with a restoration, that from the casting a model is fabricated as the duplicate preferably by pouring out of the casting with plaster, that the duplicate is attached on a ready-made base plate having references, that the base plate with the duplicate attached thereon is split apart for obtaining the duplicate sections, and thereupon the duplicate sections are measured taking into consideration references, which exist on base plate sections, onto which the duplicate sections are arranged.

3. Method according to claim 2,
**characterized in**
**that** in particular geometrical properties of the base plate and/or markings on it are used as references.

4. Method according to claim 3,
**characterized in**
**that** points and/or lines on the base plate may be used as markings.

5. Method according to claim 3,
**characterized in**
**that** as geometrical properties, delimitations such as edges or at least surface sections of the base plate are used.

6. Method according to at least claim 2,
**characterized in**
**that** the duplicate on its underside is surface-ground and is attached such as glued to a plane surface of the base plate following a tooth arc.

7. Method according to at least claim 6,
**characterized in**
**that** the duplicate is attached on the base plate in such a way, that the duplicate is spaced on all sides to the edge of the base plate.

8. Method according to at least claim 2,
**characterized in**
**that** as base plate one is used, which exhibits in or at least along a longitudinal wall running along the duplicate a texture such as a wave-shaped and/or zigzag geometry.

9. Method according to at least claim 8,
**characterized in**
**that** intersections or virtual intersections of peripheries of the wave-shaped and/or zigzag geometry are used as references.

10. Method according to at least claim 1,
**characterized by** the method steps
- Taking a casting of at least one residual tooth area comprising part of the jaw;
- Fabrication of the duplicate preferably by filling the casting with plaster;
- Mounting of the duplicate on the base plate having the references;
- Splitting of the base plate with thereon attached duplicate for obtaining model sections which comprise the duplicate sections;
- Measuring of the duplicate sections for capturing form data and the references present on the respective base plate sections;
- Match up of data, which correspond to the references of the individual model sections, with the referencing data stored in the computer; and
- Fabrication of the dental restoration under consideration of the form data and the data gained by matching.

11. Method according to claim 1,
**characterized in**
**that** the duplicate is directly provided with references.

12. Method according to claim 11,
**characterized in**
**that** the references are produced when making the casting.

## Revendications

1. Procédé de détermination de la forme d'un duplicata d'une zone de dent résiduelle à équiper d'une restauration dentaire telle qu'un bridge ou une structure, dont des sections du duplicata à équiper de la restauration et/ou déterminant sa configuration, sont détachées du duplicata et dont les données de forme à associer à leur forme sont déterminées et enregistrées dans un calculateur, pour permettre de calculer la forme de la restauration compte tenu de l'association dans l'espace des sections du duplicata l'une par rapport à l'autre,
**caractérisé en ce que**
les sections du duplicata sont déjà référencées ou vont être référencées individuellement dans leur association dans l'espace l'une par rapport à l'autre selon un référencement enregistré dans le calculateur et indépendant du duplicata.

2. Procédé selon la revendication 1,
**caractérisé en ce qu'**
on prend une empreinte d'au moins une zone de dent résiduelle de la mâchoire à traiter avec une restauration, on fabrique à partir de l'empreinte, un modèle en tant que duplicata, de préférence en coulant du plâtre dans l'empreinte, on fixe le duplicata sur un socle confectionné et présentant des références, on divise le socle avec le duplicata, fixé dessus pour obtenir les sections de duplicata, puis on mesure les sections de duplicata en tenant compte des références présentes sur des sections du socle comportant les sections de duplicata.

3. Procédé selon la revendication 2,
**caractérisé en ce que**
comme références on utilise en particulier des propriétés géométriques du socle et/ou des marques sur celui-ci.

4. Procédé selon la revendication 3,
**caractérisé en ce que**
comme marques on utilise des points et/ou des lignes sur le socle.

5. Procédé selon la revendication 3,
**caractérisé en ce qu'**
on utilise, comme propriétés géométriques, des limites telles que les arêtes ou du moins des sections superficielles du socle.

6. Procédé selon au moins la revendication 2,
**caractérisé en ce qu'**
on aplanit par polissage le dessous du duplicata et on le fixe, avec de la colle par exemple, sur une surface plane du socle suivant le parcours de l'arcade dentaire.

7. Procédé selon au moins la revendication 6,
**caractérisé en ce qu'**
on fixe le duplicata sur le socle de manière à ce qu'il soit à une certaine distance du bord du socle de tous les côtés.

8. Procédé selon au moins la revendication 2,
**caractérisé en ce qu'**
le socle utilisé présente une structuration comme une géométrie ondulée et/ou en zigzag dans ou sur au moins une paroi du côté long passant le long du duplicata.

9. Procédé selon au moins la revendication 8,
**caractérisé en ce qu'**
on utilise, comme référence, des points d'intersection ou des points d'intersection virtuels de périphéries de la géométrie ondulée et/ou en zigzag.

10. Procédé selon au moins la revendication 1,
**caractérisé par**
les étapes suivantes :
- prise d'empreinte d'au moins une partie de la mâchoire comprenant la zone de dent résiduelle à traiter,
- fabrication du duplicata, de préférence en coulant l'empreinte avec du plâtre,
- fixation du duplicata sur le socle présentant les références,
- division du socle avec le duplicata fixé dessus pour obtenir des sections de modèle présentant les sections du duplicata,
- mesure des sections du duplicata pour obtenir des données de forme et les références présentes sur les sections respectives du socle,
- appariement de données correspondant aux références des différentes sections du modèle avec les données du référencement enregistrées dans le calculateur, et
- fabrication de la restauration dentaire compte tenu des données de forme et des données obtenues grâce à l'appariement.

11. Procédé selon la revendication 1,
**caractérisé en ce qu'**
on dote immédiatement le duplicata de références.

12. Procédé selon la revendication 11,
**caractérisé en ce que**
les références sont créées lors de la fabrication d'une empreinte.
